# EUROPÄISCHE PATENTSCHRIFT

(11) **EP 2 326 322 B1**
(45) Veröffentlichungstag und Bekanntmachung des Hinweises auf die Patenterteilung: **14.10.2015**
(21) Anmeldenummer: 09778095.1
(22) Anmeldetag: 25.08.2009
(51) Int. Cl.: A61K 31/185, C07C 309/15

(54) **NEUE KRISTALLFORM VON CALCIUM-3-ACETYLAMINOPROPAN-1-SULFONAT**
NOVEL CRYSTAL FORM OF CALCIUM-3-ACETYL AMINOPROPANE-1-SULFONATE
NOUVELLE FORME CRISTALLINE DE CALCIUM-3-ACÉTYLAMINOPROPANE-1-SULFONATE

(30) Priorität: 24.09.2008 DE 102008048791
(43) Veröffentlichungstag der Anmeldung: 01.06.2011
(73) Patentinhaber: Merck Patent GmbH, 64293 Darmstadt (DE)
(72) Erfinder: MUERMANN, Antje, 64291 Darmstadt (DE); KUEHN, Clemens, 64291 Darmstadt (DE); BARTELS, Matthias, 64289 Darmstadt (DE); SAAL, Christoph, 64853 Otzberg (DE); BOSC, Nathalie, F-69250 Montanay (FR)
(86) Internationale Anmeldenummer: PCT/EP2009/006150
(87) Internationale Veröffentlichungsnummer: WO 2010/034384

(56) Entgegenhaltungen:
- CN-A- 101 492 400
- GB-A- 2 051 789
- KR-A- 20080 097 716

## Beschreibung

Die vorliegende Erfindung betrifft eine neue Kristallform von Calcium-3-Acetylaminopropan-1-sulfonat, Verfahren zu ihrer Herstellung sowie ihre Verwendung in Arzneimitteln.

Calcium-3-Acetylaminopropan-1-sulfonat, das im folgenden auch als "Wirkstoff" bezeichnet wird, hat folgende chemische Struktur

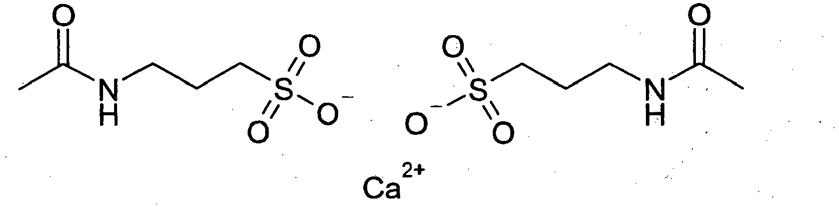

Calcium-3-Acetylaminopropan-1-sulfonat wurde erstmalig in DE 3019350 A1 beschrieben und wird als Wirkstoff zur Behandlung des Alkoholismus eingesetzt. Ebenfalls beschrieben ist die Verwendung dieses Wirkstoffs zur Behandlung des Tinnitus.

Gemäß DE 3019350 A1 ist Calcium-3-Acetylaminopropan-1-sulfonat ein kristallines farbloses Pulver mit einem Festpunkt von ungefähr 270°C und einem Wassergehalt von 3,6%, als elementare Zusammensetzung wurden 9,7% Calcium und 6,8% Stickstoff gemessen (die theoretischen Werte sind 10 % und 7%).

Toffoli et al. beschreibt die Kristallform von Calcium-3-Acetylaminopropan-1-sulfonat (P. Toffoli, N. Rodier, R. Ceolin, P. Ladure, G. Tran, Acta Crystallographica, Sect. C, Crystal Structure Communications, 1988, 44, 1493) diese Form wird nachfolgend auch als "Kristallform I" oder "Form I" bezeichnet. Gemäß Toffoli et. al. ist die Form I durch folgende Parameter charakterisiert: monoclin, P2₁/c, a = 5.4584 Å, b = 18.949 Å, c = 8.550 Å, β = 105.49°, V = 852.2 Å³.

Die in Toffoli et al. beschriebene Kristallform von Calcium-3-Acetylaminopropan-1-sulfonat entspricht der Kristallform von Calcium-3-Acetylaminopropan-1-sulfonat, wie sie in dem vermarkteten Arzneimittel enthalten ist.

Es wurde nun gefunden, dass Calcium-3-Acetylaminopropan-1-sulfonat auch in einer anderen Kristallform vorliegen kann, die nachfolgend auch "Form II" oder "Kristallform II" genannt wird. Der Festpunkt von Form II beträgt ungefähr 364°C und liegt damit deutlich höher als in DE 3019350 A1 beschrieben.

Überraschenderweise hat sich für neue gefundene Form II gezeigt, dass diese thermodynamisch stabiler ist als Form I.

Die Lösungsgeschwindigkeit von Wirkstoffen wird unter anderem von deren Kristallform bestimmt. Liegt ein Wirkstoff in verschiedenen Kristallformen vor und ist dieser als Wirkstoff für ein Arzneimittel vorgesehen, ist es vorteilhaft, wenn die thermodynamisch stabilere Form des Wirkstoffs Verwendung findet. Denn hierdurch kann grundsätzlich ausgeschlossen werden, dass sich die im Arzneimittel enthaltene thermodynamisch instabilere (metastabile) Form nach Herstellung des Arzneimittels bis zur Anwendung durch den Patienten, insbesondere während der Lagerung des Arzneimittels, in die thermodynamisch stabilere Form umwandelt.

Umwandlung von einer Kristallform in eine andere führt unter anderem zu einer Veränderung der Lösungsgeschwindigkeit des Wirkstoffs, sodass die ursprüngliche Freisetzung des Wirkstoffs aus dem Arzneimittel nicht mehr reproduzierbar ist. Veränderung der Lösungsgeschwindigkeit des Wirkstoffes hat nachteilhaft zur Folge, dass nach Einnahme des Arzneimittels durch den Patienten der in diesem enthaltene Wirkstoff im Gastrointestinaltrakt nicht mehr in der vorbestimmten Geschwindigkeit und Menge aus dem Arzneimittel freigesetzt und resorbiert wird, so dass dessen Bioverfügbarkeit und damit auch dessen Wirksamkeit nicht mehr sichergestellt ist. Somit wird die Verwendung des den Wirkstoff enthaltenden Arzneimittels insgesamt infrage gestellt.

Während der Wirkstoff nach DE 3019350 A1 Kristallwasser enthält (es sind 3,6% genannt), ist dieses in Form II nicht vorhanden.

Wie bereits erwähnt, ist die Kristallform II des Wirkstoffs thermodynamisch stabiler als dessen Kristallform I. So ergibt sich in Aufschlämmungs-Umwandlungs-Versuchen mit der Kristallform I eine Umlagerung in die Kristallform II. Weiterhin ergeben sich für die Form II mit 16.2 ± 0.2 J/g gegenüber -23.1 ± 0.2 J/g für die Form I signifikant niedrigere Lösungsenthalpien ((6.9 ± 0.4 J/g), jeweils gemessen in Wasser bei 25 °C mit einem 2225 Thermometrics Solution Calorimeter). Form II weist somit eine geringere freie Gibbs-Enthalpie auf und ist thermodynamisch stabiler als die Form I.

Form II kann hergestellt werden, indem eine Suspension von Kristallen der Form I von Calcium-3-Acetylaminopropan-1-sulfonat in einem geeigneten Suspensionsmittel gerührt und der erhaltene Rückstand abgetrennt und getrocknet wird. Als Suspensionsmittel geeignet sind Lösungsmittel, in denen der Wirkstoff löslich ist, wobei der Wirkstoff im Verhältnis zu dem jeweiligen Lösungsmittel in der Suspension in einem Gewichtsverhältnis eingesetzt wird, in dem die Löslichkeitsgrenze des Wirkstoffs in dem Lösungsmittel überschritten ist, so dass der Wirkstoff in der Suspension überwiegend als Feststoff vorliegt. Geeignete Suspensionsmittel sind zum Beispiel Wasser, Methanol und/oder Tetrahydrofuran (THF), wobei Wasser besonders bevorzugt ist.

Form I des Wirkstoffs kann gemäß dem im Stand der Technik beschriebenen Verfahren erhalten werden.

Die Kristallformen können nach Standardmethoden bestimmt werden, wie zum Beispiel beschrieben in C.W. Bunn, "Chemical Crystallography" 1948, Clarendon Press, London; oder H.P. Klug, L.E. Alexander, "X-Ray Diffraction Procedures" 1974, John Wiley and Sons, New York.

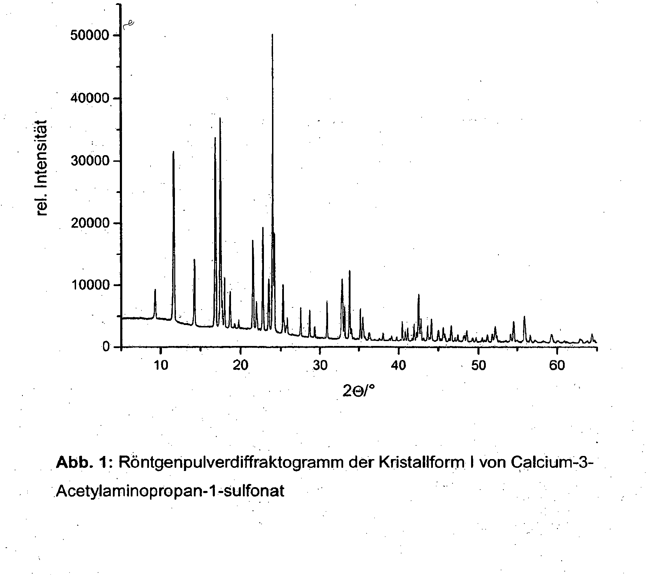

**Abb. 1** zeigt ein Röntgendiffraktogramm von Form I des Werkstoffs, die sich hieraus ergebenden Parameter sind in nachfolgender **Tab. 1** aufgeführt.

**Tab. 1: Röntgendiffraktogramm von Form I, gemessen mit Bruker D5000 Diffractometer, Transmissionsbetrieb, Generatorleistung 40 kV/30 mA, Cu-Kα1-Strahlung (λ = 1,54 Å), Ortsempfindlicher Detektor (3.3 kV), Messbereich: 3-65 °2Θ, Schrittgröße: 0,05 °2Θ, Zeit/Schritt: 1,4 s. Die Diffraktogramme werden im gesamten Aufnahmebereich 3-65 °2Θ untergrundkorrigiert und die Reflexintensitäten der stärksten Reflexe ermittelt. Die Toleranz der Winkellagen liegt bei ± 0,1 °2Θ für die verwendete Cu-Kα1-Strahlung.**

| **Nr.** | **d [Å]** | **2Θ±0.1** | **I/I₀[%]** |
|---|---|---|---|
| 1 | 9.34 | 9.3 | 18 |
| 2 | 7.61 | 11.7 | 62 |
| 3 | 6.21 | 14.2 | 27 |
| 4 | 5.30 | 16.8 | 66 |
| 5 | 5.08 | 17.5 | 71 |
| 6 | 4.93 | 118.0 | 22 |
| 7 | 4.76 | 18.7 | 18 |
| 8 | 4.49 | 19.7 | 10 |
| 9 | 4.13 | 21.6 | 34 |
| 10 | 4.06 | 21.9 | 14 |
| 11 | 3.91 | 22.7 | 38 |
| 12 | 3.77 | 23.6 | 21 |
| 13 | 3.69 | 24.0 | 100 |
| 14 | 3.53 | 25.2 | 20 |

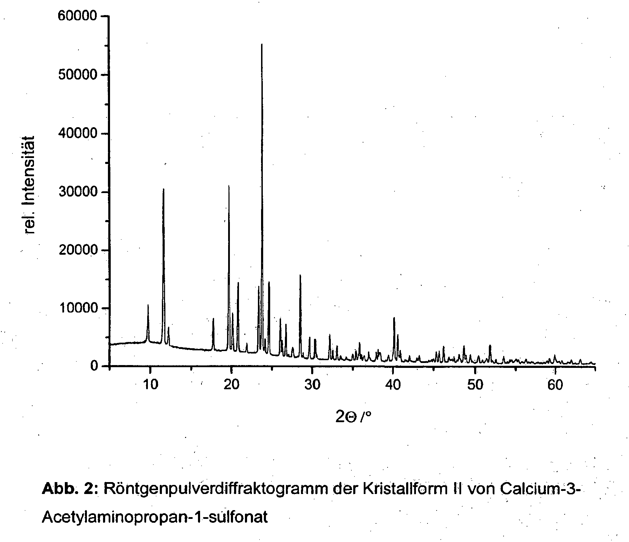

**Abb. 2** zeigt ein Röntgendiffraktogramm von Form II des Wirkstoffs, die sich hieraus ergebenden Parameter sind in nachfolgender **Tab. 2** zusammengestellt.

**Tab. 2: Röntgendiffraktogramm von Form II, Messung und Auswertung erfolgt wie zu Tab. 1 beschrieben.**

| **Nr.** | **d [Å]** | **2Θ±0.1** | **I/I₀[%]** |
|---|---|---|---|
| 1 | 7.59 | 11.6 | 55 |
| 2 | 4.51 | 19.7 | 56 |
| 3 | 4.26 | 20.8 | 26 |
| 4 | 3.80 | 23.4 | 25 |
| 5 | 3.74 | 23.8 | 100 |
| 6 | 3.61 | 24.6 | 26 |
| 7 | 3.13 | 28.5 | 28 |

In einer bevorzugten Ausführungsform ist die Form II durch Röntgendaten gemäß nachfolgender **Tab. 2a** gekennzeichnet. Die Daten gemäß **Tab. 2a** enthalten die Reflexe aus **Tab. 2** und zusätzlich 6 weitere Reflexe geringerer Intensität.

**Tab. 2a: Röntgendiffraktogramm von Form II, Messung und Auswertung erfolgt wie zu Tab. 1 beschrieben.**

| **Nr.** | **d [Å]** | **2Θ±0.1** | **I/I₀[%]** |
|---|---|---|---|
| 1 | 9.04 | 9.8 | 19 |
| 2 | 7.59 | 11.6 | 55 |
| 3 | 5.01 | 17.7 | 15 |
| 4 | 4.51 | 19.7 | 56 |
| 5 | 4.40 | 20.2 | 17 |
| 6 | 4.26 | 20.8 | 26 |
| 7 | 3.80 | 23.4 | 25 |
| 8 | 3.74 | 23.8 | 100 |
| 9 | 3.61 | 24.6 | 26 |
| 10 | 3.42 | 26.0 | 15 |
| 11 | 3.33 | 26.7 | 13 |
| 12 | 3.13 | 28.5 | 28 |
| 13 | 2.24 | 40.2 | 15 |

Gegenstand der vorliegenden Erfindung ist daher Kristallform II von Calcium-3-Acetylaminopropan-1-sulfonat, gekennzeichnet durch die in **Tab**. **2** angegebenen charakteristischen Gitterebenenabstände.

Ebenfalls Gegenstand der vorliegenden Erfindung ist Kristallform II von Calcium-3-Acetylaminopropan-1-sulfonat, gekennzeichnet durch die in **Tab**. **2a** angegebenen charakteristischen Gitterebenenabstände.

Weiterhin Gegenstand der vorliegenden Erfindung ist Kristallform II von Caicium-3-Acetylaminopropan-1-sulfonat, die durch die physikalische Daten nach mindestens einem der vor- und nachstehend beschriebenen analytischen Verfahren charakterisiert ist.

Bei Untersuchung mittels Einkristall-Röntgendiffraktometrie ergeben sich für die Kristallform II von Calcium-3-Acetylaminopropan-1-sulfonat folgende charakteristische Parameter: Triclin, *P_{1̅}*, a = 5.54 Å, b = 8.15 Å, c = 9.76, α = 69.1 °, β = 84.3°, γ = 89.4°, V = 409.66 Å³ (Oxford Xcalibur Diffractometer (Mo K_{α} Strahlen), Graphit-Monochromator, Sapphire CCD, Datenauswertung mit SHELX-97 Software Suite).

Gegenstand der vorliegenden Erfindung ist weiterhin Kristallform II von Calcium-3-Acetylaminopropan-1-sulfonat, gekennzeichnet durch folgende Parameter: *P*_{1̅}, a = 5.54 A, b = 8.15 A, c = 9.76, α = 69.1°, β = 84.3°, γ = 89.4°, V = 409.66 A³.

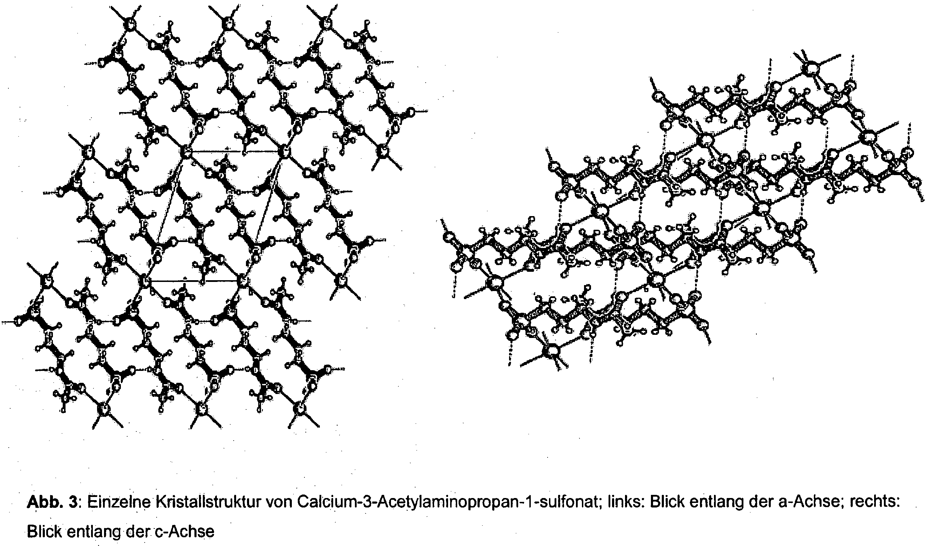

Die sich ergebende Kristallstruktur ist in **Abb. 3** dargestellt. Wie hieraus ersichtlich ist, enthält die Kristallstruktur von Form II weder Wasser noch ein sonstiges Lösungsmittel.

Unter "Form II, im wesentlichen bestehend aus Form II" wird vor- und nachstehend verstanden, dass Kristallform II weniger als 5 Gew.-%, bevorzugt weniger als 2 Gew.-% und besonders bevorzugt weniger als ein Gew.-% Form I enthält.

Gegenstand der vorliegenden Erfindung ist somit weiterhin Calcium-3-Acetylaminopropan-1-sulfonat im wesentlichen bestehend aus Kristallform II.

Gegenstand der Erfindung ist weiterhin ein Verfahren zur Herstellung der Kristallform II des Wirkstoffs, das dadurch gekennzeichnet ist, dass Kristalle von Calcium-3-Acetylaminopropan-1-sulfonat in der Kristallform I in einem Suspensionsmittel gerührt und der erhaltene Rückstand abgetrennt und getrocknet werden.

Der Wirkstoff kann natürlich auch als Mischung seiner Kristallformen II und I eingesetzt werden, wobei jeweils mehr als 10 Gew.-%,. 20 Gew.-%, 30 Gew.-%, 40 Gew.-%, 50 Gew.-%, 60 Gew.-%, 70 Gew.-%, 80 Gew.-% bzw. 90 Gew.-% der Kristallform II enthalten sein können. Erfindungsgemäß bevorzugt sind Mischungen mit mehr als 70 Gew.-%, besonders bevorzugt mit mehr als 80 Gew.-% und besonders bevorzugt mit mehr als 90 Gew.-% der Kristallform II.

Kristallform II ist, wie bereits beschrieben, thermodynamisch stabil und daher besonders geeignet als Arzneimittel. Gegenstand der Erfindung ist daher auch Kristallform II als Arzneimittel.

Ebenfalls Gegenstand der Erfindung ist eine pharmazeutische Zubereitung enthaltend die Kristallform II.

Weiterer Gegenstand der vorliegenden Erfindung ist Kristallform II zur Herstellung eines Arzneimittels.

Die Ausführungsbeispiele, ohne hierauf beschränkt zu sein, erläutern die Erfindung.

### Beispiel 1:

### Herstellung der Kristallform II von Calcium-3-Acetylaminopropan-1-sulfonat

3 g Calcium-3-Acetylaminopropan-1-sulfonat werden in 2 ml demineralisiertem Wasser suspendiert und über 7 Tage bei Raumtemperatur gerührt. Die erhaltende Suspendierung wird filtriert und getrocknet.

### Beispiel 2:

### Analytische Charakterisierung der gemäß Beispiel 1 erhaltenen Kristallform II

Zur Charakterisierung der Kristallform der nach Beispiel 1 erhaltenen Kristalle (Form II) werden Infrarot- (FT-IR) und Raman-Spektren (FT-Raman) aufgenommen. FT-IR und FT-Raman sind Standardverfahren und werden wie im Europäischen Arzneibuch, 6. Auflage, Kapitel 2.02.24 und 2.02.48 beschrieben durchgeführt.

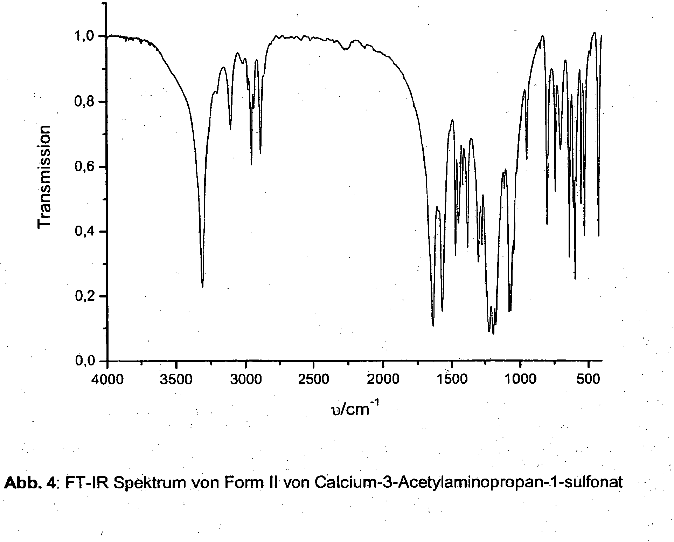

Die FT-IR Spektroskopie erfolgt mit KBr-Presslingen. Das gemessene FT-IR-Spektrum ist **in** **Abb. 4** wiedergegeben, es weist die nachfolgend aufgeführten typischen Banden auf (angegeben sind die Wellenzahlen der IR-Banden (cm⁻¹ ± 2 cm⁻¹) und deren relative Intensitäten):

3311 cm⁻¹ (m), 3106 cm⁻¹ (m), 2955 cm⁻¹ (m), 2888 cm⁻¹ (m), 1637 cm⁻¹ (s), 1568 cm⁻¹ (s), 1470 cm⁻¹ (m), 1449 cm⁻¹ (m), 1417 cm⁻¹ (m), 1380 cm⁻¹ (m), 1302 cm⁻¹ (m), 1274 cm⁻¹ (m), 1223 cm⁻¹ (s), 1191 cm⁻¹ (s), 1172 cm⁻¹ (s), 1076 cm⁻¹ (s), 1066 cm⁻¹ (s), 953 cm⁻¹ (m), 804 cm⁻¹ (m), 744 cm⁻¹ (m), 706 cm⁻¹ (m), 642 cm⁻¹ (m), 611 cm⁻¹ (m), 598 cm⁻¹ (m), 556 cm⁻¹ (m), 529 cm⁻¹ (m), 424 cm⁻¹ (m).

| | |
|---|---|
| Messbedingungen: | FT-IR-Spektroskopie, Bruker Vector 22, 2 cm⁻¹ spektrale Auflösung, 32 Scans. |
| Auswertung: | Das erhaltene FT-IR-Spektrum ist untergrundkorrigiert (Bruker OPUS Software). Die Banden sind aufgrund ihrer Transmission eingeteilt in s = stark (Transmission ≤ 20%), m = mittel (20% < Transmission ≤ 60%) w = schwach (weak) (Transmission > 60%). |

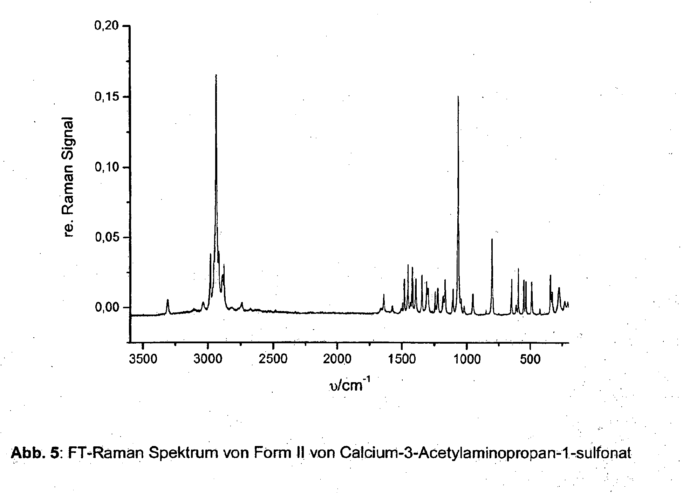

Das gemessene FT-Raman-Spektrum ist in **Abb. 5** dargestellt und hat die nachfolgend aufgeführten typischen Banden:
3310 cm⁻¹ (w), 2981 cm⁻¹ (m), 2937 cm⁻¹ (s), 2877 cm⁻¹ (m), 1637 cm⁻¹ (m), 1480 cm⁻¹ (m), 1453 cm⁻¹ (m), 1419 cm⁻¹ (m), 1392 cm⁻¹ (m), 1345 cm⁻¹ (m), 1310 cm⁻¹ (m), 1300 cm⁻¹ (w), 1244 cm⁻¹ (w), 1226 cm⁻¹ (m), 1169 cm⁻¹ (m), 1109 cm⁻¹ (m), 1070 cm⁻¹ (s), 955 cm⁻¹ (m), 805 cm⁻¹ (m), 649 cm⁻¹ (m), 595 cm⁻¹ (m), 553 cm⁻¹ (m), 534 cm⁻¹ (m), 487 cm⁻¹ (m).

Wellenzahlen der Ramanbanden (cm⁻¹ ± 2 cm⁻¹) und deren relative Intensitäten

| | |
|---|---|
| Messbedingungen: | FT-Raman-Spektroskopie, Bruker RFS 100, 1064 nm Anregung, 500 mW, 1 cm⁻¹ spektrale Auflösung, 500 Scans. |
| Auswertung: | Das erhaltene Raman-Spektrum ist im Spektralbereich 3600 - 250 cm⁻¹ vektomormiert. Die Banden sind aufgrund ihrer Intensität eingeteilt in s = stark (rel. Raman-Intensität ≥ 0,1) m = mittel (0,1 < rel. Raman-Intensität ≥ 0,025) w = schwach (weak) (rel. Raman-Intensität < 0,025). |

### Beispiel 3:

### Analytische Charakterisierung von Form I

Zu Vergleichszwecken wird die Form I des Wirkstoffs (Ausgangsmaterial von Beispiel 1) analog zu Beispiel 2 analytisch charakterisiert und ausgewertet.

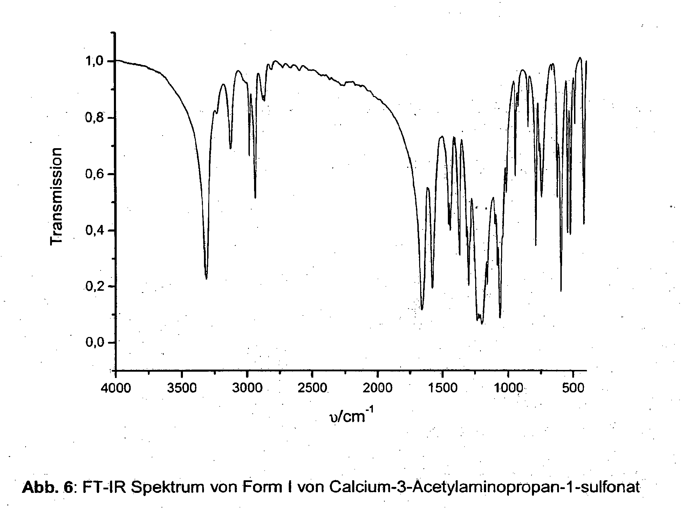

Das FT-IR Spektrum von Form I ist in **Abb. 6** dargestellt, dieses weist die folgenden Parameter auf:
3312 cm⁻¹ (m), 3123 cm⁻¹ (w), 2980 cm⁻¹ (w), 2937 cm⁻¹ (m), 2878 cm⁻¹ (w), 2864 cm⁻¹ (w), 1658 cm⁻¹ (s), 1577 cm⁻¹ (s), 1453 cm⁻¹ (m), 1441 cm⁻¹ (m), 1370 cm⁻¹ (m), 1298 cm⁻¹ (s), 1234 cm⁻¹ (s), 1219 cm⁻¹ (s), 1199 cm⁻¹ (s), 1160 cm⁻¹ (s), 1083 cm⁻¹ (m), 1064 cm⁻¹ (s), 1018 cm⁻¹ (m), 946 cm⁻¹ (m), 850 cm⁻¹ (w), 789 cm⁻¹ (m), 744 cm⁻¹ (m), 625 cm⁻¹ (m), 596 cm⁻¹ (s), 545 cm⁻¹ (m), 521 cm⁻¹ (m), 490 cm⁻¹ (w), 418 cm⁻¹ (m).

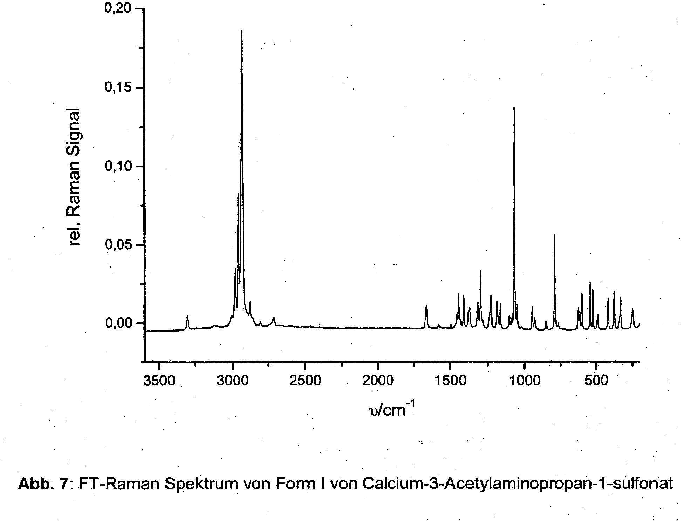

Das FT-Raman Spektrum von Form I ist in **Abb. 7** dargestellt, es ist durch die folgenden Parameter charakterisiert:
3304 cm⁻¹ (w), 2980 cm⁻¹ (m), 2958 cm⁻¹ (m), 2934 cm⁻¹ (s), 2879 cm⁻¹ (w), 1661 cm⁻¹ (w), 1454 cm⁻¹ (w), 1442 cm⁻¹ (w), 1408 cm⁻¹ (w), 1369 cm⁻¹ (w), 1316 cm⁻¹ (w), 1298 cm⁻¹ (m), 1227 cm⁻¹ (w), 1187 cm⁻¹ (w), 1165 cm⁻¹ (w), 1103 cm⁻¹ (w), 1084 cm⁻¹ (w), 1068 cm⁻¹ (s), 1051 cm⁻¹ (w), 946 cm⁻¹ (w), 929 cm⁻¹ (w), 790 cm⁻¹ (m), 627 cm⁻¹ (w), 616 cm⁻¹ (w), 598 cm⁻¹ (w), 542 cm⁻¹ (m), 524 cm⁻¹ (m), 490 cm⁻¹ (w).

## Patentansprüche

1. Kristallform II von Calcium-3-Acetylaminopropan-1-sulfonat, **gekennzeichnet durch** folgende charakteristische Gitterebenenabstände in Å:
| **Nr.** | **d [Å]** | **2Θ±0.1** |
|---|---|---|
| 1 | 7.59 | 11.6 |
| 2 | 4.51 | 19.7 |
| 3 | 4.26 | 20.8 |
| 4 | 3.80 | 23.4 |
| 5 | 3.74 | 23.8 |
| 6 | 3.61 | 24.6 |
| 7 | 3.13 | 28.5 |

2. Kristallform II von Calcium-3-Acetylaminopropan-1-sulfonat gemäß Anspruch 1, **gekennzeichnet durch** folgende charakteristische Gitterebenenabstände in Å:
| **Nr.** | **d [Å]** | **2Θ±0.1** |
|---|---|---|
| 1 | 9.04 | 9.8 |
| 2 | 7.59 | 11.6 |
| 3 | 5.01 | 17.7 |
| 4 | 4.51 | 19.7 |
| 5 | 4.40 | 20.2 |
| 6 | 4.26 | 20.8 |
| 7 | 3.80 | 23.4 |
| 8 | 3.74 | 23.8 |
| 9 | 3.61 | 24.6 |
| 10 | 3.42 | 26.0 |
| 11 | 3.33 | 26.7 |
| 12 | 3.13 | 28.5 |
| 13 | 2.24 | 40.2 |

3. Calcium-3-Acetylaminopropan-1-sulfonat enthaltend Kristallform II nach Anspruch 1 und/oder 2.

4. Calcium-3-Acetylaminopropan-1-sulfonat bestehend aus Kristallform II nach Anspruch 1 und/oder 2.

5. Verfahren zur Herstellung der Kristallform II nach Anspruch 1 und/oder 2, **dadurch gekennzeichnet, dass** Kristalle von Calcium-3-Acetylaminopropan-1-sulfonat in der Kristallform I, **gekennzeichnet durch** folgende charakteristische Gitterebenenabstände in A:
| **Nr.** | **d [Å]** | **2Θ±0.1** | **I/I₀[%]** |
|---|---|---|---|
| 1 | 9.34 | 9.3 | 18 |
| 2 | 7.61 | 11.7 | 62 |
| 3 | 6.21 | 14.2 | 27 |
| 4 | 5.30 | 16.8 | 66 |
| 5 | 5.08 | 17.5 | 71 |
| 6 | 4.93 | 18.0 | 22 |
| 7 | 4.76 | 18.7 | 18 |
| 8 | 4.49 | 19.7 | 10 |
| 9 | 4.13 | 21.6 | 34 |
| 10 | 4.06 | 21.9 | 14 |
| 11 | 3.91 | 22.7 | 38 |
| 12 | 3.77 | 23.6 | 21 |
| 13 | 3.69 | 24.0 | 100 |
| 14 | 3.53 | 25.2 | 20 |
in einem Suspensionsmittel, wobei das Suspensionsmittel aus Wasser, Methanol und/oder Tetrahydrofuran (THF) ausgewählt ist, gerührt und der erhaltene Rückstand abgetrennt und getrocknet werden.

6. Kristallform nach einem oder mehreren der vorstehenden Ansprüche als Arzneimittel.

7. Pharmazeutische Zubereitung enthaltend die Kristallform II nach einem öder mehreren der vorstehenden Ansprüche und einen oder mehrere Hilfsstoffe.

8. Verwendung der Kristallform II gemäß einem oder mehreren der vorstehenden Ansprüche zur Herstellung eines Arzneimittels.

## Claims

1. Crystal form II of calcium 3-acetylaminopropane-1-sulfonate, **characterised by** the following characteristic lattice plane separations in Å:
| **No.** | **d [Å]** | **2Θ±0.1** |
|---|---|---|
| 1 | 7.59 | 11.6 |
| 2 | 4.51 | 19.7 |
| 3 | 4.26 | 20.8 |
| 4 | 3.80 | 23.4 |
| 5 | 3.74 | 23.8 |
| 6 | 3.61 | 24.6 |
| 7 | 3.13 | 28.5 |

2. Crystal form II of calcium 3-acetylaminopropane-1-sulfonate according to Claim 1, **characterised by** the following characteristic lattice plane separations in Å:
| **No.** | **d[Å]** | **2Θ±0.1** |
|---|---|---|
| 1 | 9.04 | 9.8 |
| 2 | 7.59 | 11.6 |
| 3 | 5.01 | 17.7 |
| 4 | 4.51 | 19.7 |
| 5 | 4.40 | 20.2 |
| 6 | 4.26 | 20.8 |
| 7 | 3.80 | 23.4 |
| 8 | 3.74 | 23.8 |
| 9 | 3.61 | 24.6 |
| 10 | 3.42 | 26.0 |
| 11 | 3.33 | 26.7 |
| 12 | 3.13 | 28.5 |
| 13 | 2.24 | 40.2 |

3. Calcium 3-acetylaminopropane-1-sulfonate comprising crystal form II according to Claim 1 and/or 2.

4. Calcium 3-acetylaminopropane-1-sulfonate consisting of crystal form II according to Claim 1 and/or 2.

5. Process for the preparation of crystal form II according to Claim 1 and/or 2, **characterised in that** crystals of calcium 3-acetylaminopropane-1-sulfonate in crystal form I, **characterised by** the following characteristic lattice plane separations in Å:
| **No.** | **d [Å]** | **2Θ±0.1** | **I/I₀[%]** |
|---|---|---|---|
| 1 | 9.34 | 9.3 | 18 |
| 2 | 7.61 | 11.7 | 62 |
| 3 | 6.21 | 14.2 | 27 |
| 4 | 5.30 | 16.8 | 66 |
| 5 | 5.08 | 17.5 | 71 |
| 6 | 4.93 | 18.0 | 22 |
| 7 | 4.76 | 18.7 | 18 |
| 8 | 4.49 | 19.7 | 10 |
| 9 | 4.13 | 21.6 | 34 |
| 10 | 4.06 | 21.9 | 14 |
| 11 | 3.91 | 22.7 | 38 |
| 12 | 3.77 | 23.6 | 21 |
| 13 | 3.69 | 24.0 | 100 |
| 14 | 3.53 | 25.2 | 20 |
are stirred in a suspension medium, where the suspension medium is selected from water, methanol and/or tetrahydrofuran (THF), and the residue obtained is separated off and dried.

6. Crystal form according to one or more of the preceding claims as medicament.

7. Pharmaceutical composition comprising crystal form II according to one or more of the preceding claims and one or more assistants.

8. Use of crystal form II according to one or more of the preceding claims for the preparation of a medicament.

## Revendications

1. Forme cristalline II de 3-acétylaminopropane-1-sulfonate de calcium, **caractérisée par** les séparations de plan de réseau caractéristiques suivantes en Å:
| **n°** | **d [Å]** | **2Θ±0,1** |
|---|---|---|
| 1 | 7,59 | 11,6 |
| 2 | 4,51 | 19,7 |
| 3 | 4,26 | 20,8 |
| 4 | 3,80 | 23,4 |
| 5 | 3,74 | 23,8 |
| 6 | 3,61 | 24,6 |
| 7 | 3,13 | 28,5 |

2. Forme cristalline II de 3-acétylaminopropane-1-sulfonate de calcium selon la revendication 1, **caractérisée par** les séparations de plan de réseau caractéristiques suivantes en Å:
| **n°** | **d [Å]** | **2Θ±0,1** |
|---|---|---|
| 1 | 9,04 | 9,8 |
| 2 | 7,59 | 11,6 |
| 3 | 5,01 | 17,7 |
| 4 | 4,51 | 19,7 |
| 5 | 4,40 | 20,2 |
| 6 | 4,26 | 20,8 |
| 7 | 3,80 | 23,4 |
| 8 | 3,74 | 23,8 |
| 9 | 3,61 | 24,6 |
| 10 | 3,42 | 26,0 |
| 11 | 3,33 | 26,7 |
| 12 | 3,13 | 28,5 |
| 13 | 2,24 | 40,2 |

3. 3-Acétylaminopropane-1-sulfonate de calcium comprenant la forme cristalline II selon la revendication 1 et/ou 2.

4. 3-Acétylaminopropane-1-sulfonate de calcium constitué de la forme cristal-line II selon la revendication 1 et/ou 2.

5. Procédé de préparation de la forme cristalline II selon la revendication 1 et/ou 2, **caractérisé en ce que** des cristaux de 3-acétylaminopropane-1-sulfonate de calcium sous la forme cristalline I, **caractérisée par** les séparations de plan de réseau caractéristiques suivantes en Å:
| **n°** | **d [Å]** | **2Θ±0,1** | **I/I₀[%]** |
|---|---|---|---|
| 1 | 9,34 | 9,3 | 18 |
| 2 | 7,61 | 11,7 | 62 |
| 3 | 6,21 | 14,2 | 27 |
| 4 | 5,30 | 16,8 | 66 |
| 5 | 5,08 | 17,5 | 71 |
| 6 | 4,93 | 18,0 | 22 |
| 7 | 4,76 | 18,7 | 18 |
| 8 | 4,49 | 19,7 | 10 |
| 9 | 4,13 | 21,6 | 34 |
| 10 | 4,06 | 21,9 | 14 |
| 11 | 3,91 | 22,7 | 38 |
| 12 | 3,77 | 23,6 | 21 |
| 13 | 3,69 | 24,0 | 100 |
| 14 | 3,53 | 25,2 | 20 |
sont agités dans un milieu de suspension, où le milieu de suspension est choisi parmi l'eau, le méthanol et/ou le tétrahydrofurane (THF), et le résidu obtenu est séparé et séché.

6. Forme cristalline selon l'une ou plusieurs parmi les revendications précédentes, comme médicament.

7. Composition pharmaceutique comprenant une forme cristalline Il selon l'une ou plusieurs parmi les revendications précédentes, et un ou plusieurs auxiliaires.

8. Utilisation d'une forme cristalline Il selon l'une ou plusieurs parmi les revendications précédentes, pour la préparation d'un médicament.
